(19) Europäisches Patentamt
European Patent Office
Office européen des brevets

(11) Veröffentlichungsnummer: **0 008 358**
**B1**

(12) **EUROPÄISCHE PATENTSCHRIFT**

(45) Veröffentlichungstag der Patentschrift: **11.05.83**

(21) Anmeldenummer: **79102431.8**

(22) Anmeldetag: **13.07.79**

(51) Int. Cl.[3]: **C 07 D 307/60,**
C 07 D 303/48,
C 07 D 301/02 //A23L1/226

# (54) Neue Bis-epoxy-dialkoxy-alkane, deren Herstellung und Verwendung.

(30) Priorität: **19.07.78 DE 2831676**

(43) Veröffentlichungstag der Anmeldung:
**05.03.80 Patentblatt 80/5**

(45) Bekanntmachung des Hinweises auf die Patenterteilung:
**11.05.83 Patentblatt 83/19**

(84) Benannte Vertragsstaaten:
**CH DE FR GB NL**

(56) Entgegenhaltungen:
**DE - A - 1 768 649**
**DE - A - 1 915 788**
**DE - A - 2 359 891**
**DE - A - 2 845 843**
**DE - C - 2 105 014**

**HOUBEN-WEYL, "Methoden der organischen Chemie" 4. Auflage, Band VI, Teil 3, 1965 THIEME VERLAG, Stuttgart**

(73) Patentinhaber: **BASF Aktiengesellschaft**
**Carl-Bosch-Strasse 38**
**D-6700 Ludwigshafen (DE)**

(72) Erfinder: **Baumann, Manfred, Dr. Dipl.-Chem.**
**Im Sennteich 26**
**D-6800 Mannheim 24 (DE)**
Erfinder: **Hoffmann, Werner, Dr.Dipl.-Chem.**
**Ringstrasse 11 C**
**D-6701 Neuhofen (DE)**

**EP 0 008 358 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Neue Bis-epoxy-dialkoxy-alkane, deren Herstellung und Verwendung

Die Erfindung betrifft die neuen Bis-epoxy-dialkoxy-alkane der allgemeinen Formel I

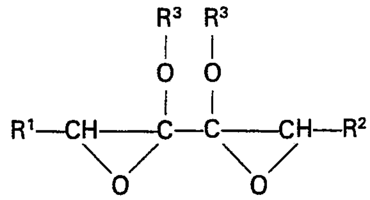

(I),

in der $R^1$ bis $R^3$ Alkyl mit 1 bis 4 C-Atomen, vorzugsweise Methyl oder Äthyl bedeuten; ein Verfahren zu deren Herstellung sowie deren Verwendung zur Herstellung von Aromastoffen der allgemeinen Formel III

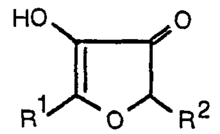

(III),

in der $R^1$ und $R^2$ die oben angegebene Bedeutung haben, insbesondere die Verwendung zur Herstellung des begehrten Aromastoffs 2,5-Dimethyl-4-hydroxy-2,3-dihydro-furan-3-on der Formel IIIa:

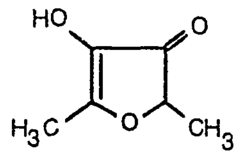

(IIIa)

Die neuen Bis-epoxy-dialkoxy-alkane sind selbst nicht als Riechstoffe verwendbar, sie erlangen jedoch besondere Bedeutung als wertvolle Vorprodukte für die Herstellung der Aromastoffe der Formel III, insbesondere des Aromastoffs der Formel IIIa.

Die Synthese des natürlichen, in Ananas und Erdbeeren vorkommenden Fruchtaromastoffes der Formel IIIa ist Gegenstand zahlreicher Verfahrensvorschriften, die jedoch aus verschiedenen Gründen unbefriedigend sind und sich auf wirtschaftliche Weise nicht befriedigend in den technischen Maßstab übertragen lassen.

So geht man beispielsweise nach der Arbeit von Büchi und Demole (J. Org. Chem., Band 38, 1973, Seite 123 f) von 2,5-Dimethyl-furanon aus, überführt dieses durch Bromierung in methanolischer Lösung in das 2,5-Dimethyl-2,5-dimethoxy-2,5-dihydrofuran und oxidiert die letztgenannte Verbindung mit Kaliumchlorat und katalytischen Mengen Osmium-tetroxid zum 3,4-Dihydroxy-hexan-2,5-dion, welches sich in Gegenwart von Basen zur Verbindung IIIa cyclisieren läßt. Diese Synthese ist umständlich und erfordert die Verwendung des nicht unproblematischen Kaliumchlorates und des teuren und giftigen Osmiumtetroxides, so daß sie für technische Zwecke nicht in Betracht kommt.

Ebenso unbefriedigend ist eine von den gleichen Autoren (loc. cit.) beschriebene Variante dieses Verfahrens, bei der lediglich das 3,4-Dihydroxy-hexan-2,5-dion auf einem anderen Wege, nämlich durch Hydrodimerisierung von Methyl-glyoxal in Gegenwart von Zinkstaub hergestellt wird. Diese Reaktion, die aus vielen Teiloperationen besteht, ist verfahrenstechnisch in größeren Ansätzen nur schwer zu beherrschen und liefert überdies nur Ausbeuten von rund 20%.

Auch das Verfahren der DE-PS 2 105 014, nach welchem 2,5-Dihalogen-hexan-3,4-dione mit wäßrigem Alkali zu IIIa cyclisiert werden, läßt zu wünschen übrig, da man hierbei nur Ausbeuten von etwa 30% erzielt.

In einem ähnlichen Verfahren (DE-OS 1 768 649) wird 2,5-Dihydroxy-hexan-3,4-dion zu IIIa umgesetzt. Da dieses Dioldion jedoch seinerseits nur durch die gefährliche und technisch schwer zu beherrschende Ozonisierung von 2,5-Dihydroxy-hex-3-in hergestellt wird, läßt sich auch dieses Verfahren technisch nicht ohne große Schwierigkeiten realisieren.

Anstelle der Diol-Dione wurden auch bereits deren Ester, wie die Diacetate zur Cyclisierung zu IIIa eingesetzt (vgl. DE-OS 1 915 788). Die Herstellung dieser Ester verläuft jedoch über eine Grignard-Reaktion und eine Oxydation mit Kaliumpermanganat, d.h. Verfahrensschritte, die bekanntlich eine exakte Einhaltung ganz bestimmter Reaktionsbedingungen erfordern, wodurch eine technische Synthese schwerfällig und teuer wird.

Bei einem weiteren Verfahren (DE-OS 2 359 891), das über die Kondensation von einem Natriumacetessigester mit $\alpha$-Brom-propionylchlorid und anschließende Decarboxylierung und Oxydation verläuft, werden nur Ausbeuten von etwa 15%, bezogen auf das Brompropionylchlorid, erzielt.

Aufgabe der Erfindung war daher, neue Wege und neue Ausgangsverbindungen zu finden, mit deren Hilfe es gelingt, den begehrten Aromastoff IIIa auf wirtschaftlichere und verfahrenstechnisch einfachere Weise herzustellen als bisher.

Mit den Bis-epoxy-dialkoxy-alkanen der allgemeinen Formel I

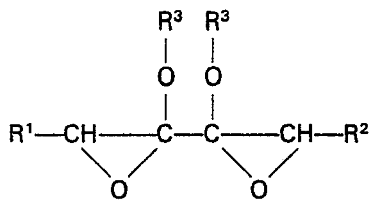

(I),

in der $R^1$ bis $R^3$ Alkyl mit 1—4 C-Atomen, vorzugsweise Methyl oder Äthyl, insbesondere Methyl bedeuten, wurden neue Verbindungen gefunden, die einerseits auf relativ einfache Weise in guten Ausbeuten hergestellt werden können und durch Erhitzen mit starken Säuren auf einfache Weise und mit relativ guten Ausbeuten in die Verbindung IIIa bzw. allgemein in die Aromastoffe III überführt werden können.

Gegenstand der Erfindung ist daher neben den neuen Bis-epoxy-dialkoxy-alkanen der Formel I ein Verfahren zur Herstellung der neuen Bis-epoxy-dialkoxy-alkane der allgemeinen Formel I, das dadurch gekennzeichnet ist, daß man $\alpha,\alpha'$-Dihalogen-1,2-diketone der allgemeinen Formel II

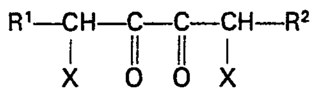

(II),

in der $R^1$ und $R^2$ die oben angegebene Bedeutung haben und X für Cl oder Br steht, bei Temperaturen von —20 bis +100°C, vorzugsweise 0 bis 25°C mit einem Alkalihydroxid oder einem Alkalialkoxid der Formel $R^3$—O—Me, in der Me für K, Na oder Li, vorzugsweise Na, steht und einem Alkohol der Formel $R^3$—OH umsetzt.

Gegenstand der Erfindung ist ferner die Verwendung der neuen Bis-epoxy-dialkoxy-alkane der allgemeinen Formel I zur Herstellung von Aromastoffen der allgemeinen Formel III

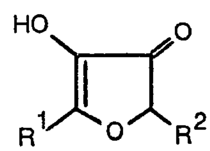

(III),

in der $R^1$ und $R^2$ die oben angegebene Bedeutung haben, durch Erhitzen mit starken Mineralsäuren oder starken organischen Säuren auf Temperaturen von 60 bis 120°C.

Die als Ausgangsverbindungen zur verwendenden $\alpha,\alpha'$-Dihalogen-1,2-diketone der allgemeinen Formel II können auf relativ einfache und bekannte Weise durch Halogenierung der entsprechenden Diketone mit der annähernd stöchiometrischen Menge des betreffenden Halogens unter sauren Bedingungen hergestellt werden.

Als Alkalihydroxide kommen KOH, NaOH und Li OH, vorzugsweise NaOH in Betracht.

Als Alkalialkoxide werden Kalium-, Natrium- oder Lithiumalkoxide mit 1 bis 4 C-Atomen, vorzugsweise Natriumalkoxide, insbesondere Natriummethylat, verwendet.

Die Alkalihydroxide bzw. Alkalialkoxide verwendet man im allgemeinen in Mengen von 2 bis 4 Mol, vorzugsweise 2 bis 2,5 Mol pro Mol Dihalogenid.

Als Alkanole eignen sich Alkanole mit 1 bis 4 C-Atomen, wie Methanol, Äthanol, Propanol, Isopropanol, n-Butanol und Isobutanol, insbesondere Methanol.

Bei Verwendung von Alkalialkoxiden verwendet man zweckmäßig das Alkanol, das auch dem Alkalialkoxid zugrunde liegt.

Die Alkanole verwendet man im allgemeinen in Mengen von 5 bis 20 Mol pro Mol Dihalogenid, d.h. man verwendet das Alkanol gleichzeitig als Lösungsmittel. So arbeitet man in ca. 10- bis 30%iger Lösung des Alkalihydroxides oder Alkalialkoxides. Beim Arbeiten in konzentrierteren Lösungen können durch ausfallende Salze Rührschwierigkeiten auftreten.

Die Reaktionstemperatur kann —20 bis ca. +100°C, vorzugsweise 0 bis 25°C betragen.

Die Reaktionszeit beträgt im allgemeinen 2 bis 24, vorzugsweise 3 bis 6 Stunden.

Zur Durchführung der Umsetzung geht man mit Vorteil so vor, daß man das Alkalihydroxid oder ein Alkalimetall in dem Alkanol löst und zu der erhaltenen Lösung (bzw. bei LiOH Suspension) des Alkalihydroxids bzw. -alkoxids langsam bei der Reaktionstemperatur eine Lösung des Dihalogenids der Formel II in dem entsprechenden Alkanol zugibt und das Reaktionsgemisch noch für den Rest der Reaktionszeit unter Rühren bei Raumtemperatur oder der Reaktionstemperatur beläßt. Man kann auch

die Lösung des Alkalihydroxids bzw. Alkalialkoxides zu der alkanolischen Lösung des Dihalogenids zufügen. Allerdings sind bei dieser Variante die erhaltenen Ausbeuten etwas geringer.

Die Isolierung der Bis-epoxy-dialkoxy-alkane erfolgt in an sich bekannter Weise, z.B. durch Abfiltrieren des bei der Reaktion gebildeten Salzes, Einengen der alkoholischen Lösung und anschließende Destillation oder aber durch Einengen des Reaktionsgemisches, Aufnehmen in einem mit Wasser nicht mischbaren Lösungsmittel wie Äther, Chloroform, Essigester oder dergleichen, Auswaschen des bei der Reaktion gebildeten Salzes, Trocknen, Einengen und anschließendes Destillieren.

Zur Herstellung der Aromastoffe der Formel III verwendet man die Bis-epoxy-dialkoxy-alkane jedoch mit Vorteil gleich in Form eines Rohproduktes, wie es beispielsweise durch Filtration und Einengen des Ausreagierten Reaktionsgemisches erhalten wird.

Zur Cyclisierung der Bis-epoxy-dialkoxy-alkane zu den Aromastoffen der Formel III kann jede herkömmliche starke Säure mit einer Dissozationskonstanten (in Wasser bei 25°C) von mehr als etwa $10^{-4}$ verwendet werden. Bevorzugte starke Säuren sind anorganische Säuren wie Schwefelsäure, Chlorwasserstoffsäure, Perchlorsäure, Bromwasserstoffsäure, Jodwasserstoffsäure, Phosphorsäure und dgl. Bevorzugt sind ebenfalls starke organische Säuren wie Ameisensäure, Oxalsäure, Trichloressigsäure und die Sulfonsäuren. Von den vorhergehend erwähnten starken Säuren sind in dem erfindungsgemäßen Verfahren insbesondere die relativ billigen starken anorganischen Säuren wie Schwefelsäure und Phosphorsäure, sowie die relativ wenig korrosiven starken organischen Säuren wie Oxalsäure und p-Toluolsulfonsäure bevorzugt.

Die Cyclisierung wird in Wasser oder Gemischen von Wasser mit hydrophilen Lösungsmitteln, z.B. in Gemischen von Wasser und Methanol oder Äthanol, Dioxan, Tetrahydrofuran und Dimethoxyäthan durchgeführt. Bevorzugte Lösungsmittel sind reines Wasser sowie Gemische von Methanol und Wasser oder Äthanol und Wasser.

Die Reaktionstemperaturen liegen bei 60 bis 120°C, vorzugsweise 80 bis 100°C; die Reaktionszeit je nach Temperatur und Säuremenge zwischen 1 und 12 Stunden.

Die Isolierung der Aromastoffe III erfolgt auf bekannte Art, beispielsweise durch Extraktion mit einem mit Wasser nicht oder nur wenig mischbaren Lösungsmittel, Trocknen und Einengen mit nachfolgender Destillation bzw. Sublimation.

Die erfindungsgemäßen Bis-epoxy-dialkoxy-alkane eröffnen einen überraschend vorteilhaften Weg zur synthetischen Herstellung des begehrten Aromastoffes der Formel IIIa und entsprechenden höher alkylsubstituierten Verbindungen mit sehr guten organoleptischen Eigenschaften.

Beispiel 1

Zu 80 g einer 30%igen $NaOCH_3$-Lösung (0,44 Mol) wurde innerhalb von 6 Minuten bei Temperaturen von 15 bis 20°C (d.h. bei leichter Kühlung) die Lösung von 32 g (0,175 Mol) 2,5-Dichlor-hexan-3,4-dion in 50 ml Methanol zugetropft. Anschließend wurde das Reaktionsgemisch noch 24 Stunden bei Raumtemperatur gerührt.

Zur Aufarbeitung des Reaktionsgemisches wurde das Methanol abgezogen, der Rückstand in Äther aufgenommen, wonach die gebildeten Salze mit Wasser ausgewaschen wurden. Die ätherische Lösung wurde getrocknet und eingeengt. Es hinterblieben 17,3 g entsprechend einer Rohausbeute von 57% der Theorie. Eine Probe wurde destilliert. Das neue 2,3; 4,5-Bis-epoxy-3,4-dimethoxy-hexan hat einen Siedepunkt Kp = 25—30°C bei 0,05 mbar und einem Brechungsindex $n_D^{25} = 1,4366$.

IR- und NMR-Spektrum sowie die Analyse bestätigen die Struktur. Es liegt ein Diastereomerengemisch vor.

Beispiel 2

Zu 90 g einer 30%igen $NaOCH_3$-Lösung (0,5 Mol) wurde innerhalb von 2 Stunden bei Temperaturen von 0 bis 5°C die Lösung von 55 g (0,2 Mol) 2,5-Dibrom-hexan-3,4-dion in 100 ml Methanol zugetropft. Anschließend wurde das Reaktionsgemisch noch 20 Stunden bei 0°C gerührt.

Bei der Aufarbeitung analog Beispiel 1 hinterblieben 35,5 g an rohem 2,3; 4,5-Bis-epoxy-3,4-dimethoxy-hexan (entsprechend einer 100%igen Rohausbeute).

Beispiel 3

Zu 90 g einer 30%igen $NaOCH_3$-Lösung wurde innerhalb von 2 Stunden unter Erhitzen zum Sieden unter Rückfluß die Lösung von 55 g (0,2 Mol) 2,5-Dibrom-hexan-3,4-dion in 100 ml Methanol zugetropft und das Reaktionsgemisch noch weitere 3 Stunden unter Rückfluß zum Sieden erhitzt.

Bei der Aufarbeitung analog Beispiel 1 erhielt man das 2,3; 4,5-Bis-epoxy-3,4-dimethoxy-hexan in einer Rohausbeute von 70%.

Beispiel 4

Zu 90 g einer 30%igen $NaOCH_3$-Lösung (0,5 Mol) wurde innerhalb von 2 Stunden bei Temperaturen von etwa 15 bis 20°C die Lösung von 55 g (0,2 Mol) 2,5-Dibrom-hexan-3,4-dion in 100 ml Methanol zugetropft und das Reaktionsgemisch noch 3 Stunden bei 15 bis 20°C gerührt. Bei der Aufarbeitung analog Beispiel 1 erhielt man das 2,3; 4,5-Bis-epoxy-dimethoxy-hexan in einer Rohausbeute von 91%.

Beispiel 5

5 g (0,22 Mol) Na wurden mit 110 ml Äthanol in eine Naäthylatlösung überführt, die innerhalb von 30 Minuten bei 15 bis 20°C mit einer Lösung aus 27,2 g (0,1 Mol) 2,5-Dibrom-hexan-3,4-dion in 50 ml Äthanol versetzt wurde. Anschließend wurde das Reaktionsgemisch noch 16 Stunden bei Raumtemperatur nachgerührt. Die Aufarbeitung des Reaktionsgemisches erfolgte analog Beispiel 1. Es wurden 14,9 g Rohprodukt erhalten, aus dem durch Destillation 11,5 g (entsprechend einer Ausbeute von 57% der Theorie) an 2,3; 4,5-Bis-epoxy-diäthoxy-hexan vom Siedepunkt Kp = 38—39°C bei 0,05 mbar und Brechungsindex $n_D^{25} = 1,4248$ erhalten wurden.

IR- und NMR-Spektrum sowie Analysen bestätigen die Struktur.

Beispiel 6

Aus 8,9 g (0,22 Mol) Kalium und 150 ml Isobutanol wurde eine Kaliumisobutylatlösung hergestellt. Zu dieser Lösung wurden innerhalb von 30 Minuten bei 15 bis 20°C die Lösung von 27,2 g (0,1 Mol) 2,5-Dibrom-hexan-3,4-dion in 100 ml Isobutanol getropft und das Reaktionsgemisch noch 20 Stunden bei Raumtemperatur gerührt. Die Aufarbeitung erfolgte analog Beispiel 1. Es hinterblieben 20,8 g Rohprodukt, dessen IR- und NMR-Spektren die angenommene Struktur stützen. Bei dem Versuch dieses Produkt zu destillieren, erfolgte Zersetzung.

Beispiel 7

a) 40 g (0,74 Mol) KOH wurden mit 200 ml Methanol in eine K-methylatlösung überführt, die bei 15 bis 20°C innerhalb von 60 Minuten mit einer Lösung aus 82 g (0,3 Mol) 2,5-Dibrom-hexan-3,4-dion in 100 ml Methanol versetzt wurde. Danach wurde das Reaktionsgemisch noch 4 Stunden bei 20°C gerührt.

Anschließend wurde filtriert und eingeengt. Der Rückstand wurde in Äther aufgenommen, die Salze ausgewaschen, die ätherische Lösung getrocknet und eingeengt. Es hinterblieben 34 g Rückstand, der nach IR- und NMR-Spektren neben der im Beispiel 1 erhaltenen Verbindung noch ca. 15% einer Substanz enthielt, bei der der Epoxyäther zur $\alpha$-Hydroxycarbonylverbindung geöffnet ist.

(b) Arbeitete man wie in Beispiel 7a) beschrieben, verwendete jedoch anstelle von 40 g (0,74 Mol) KOH in 200 ml Methanol 29,5 g (0,74 Mol) NaOH in 300 Methanol, so hinterblieben 24 g eines Rückstandes, der mit der gemäß Beispiel 1 erhaltenen Substanz identisch ist.

(c) Arbeitete man wie in Beispiel 7a) beschrieben, verwendete jedoch anstelle von 40 g (0,74 Mol) KOH 20 g (0,74 Mol) LiOH, so hinterblieben 14,4 g eines Rückstandes, der das gleiche Spektrum aufwies, wie der gemäß 7a) erhaltene Rückstand.

Beispiel 8

Zu einer Lösung enthaltend 0,416 Mol $NaOCH_3$ in 100 ml Methanol wurde innerhalb von 6 Minuten bei 15 bis 20°C die Lösung von 55 g (0,192 Mol) 2,5-Dibrom-heptan-3,4-dion in 30 ml Methanol zugetropft. Anschließend wurde das Reaktionsgemisch noch 16 Stunden gerührt.

Bei der Aufarbeitung des Reaktionsgemisches analog Beispiel 1 erhielt man 22,4 g eines Rückstandes. Anschließende Destillation des erhaltenen Rohproduktes ergab 17 g (entsprechend einer Ausbeute von 47% der Theorie) an 2,3; 4,5-Bis-epoxy-3,4-dimethoxy-heptan vom Siedepunkt Kp = 40—42°C bei 0,2 mbar und Brechungsindex $n_D^{25} = 1,4293$. IR- und NMR-spektroskopische Daten bestätigten die Struktur.

Beispiel 9

(a) 31 g (0,178 Mol) eines gemäß Beispiel 1 erhaltenen Roh-2,3; 4,5-Bis-epoxy-3,4-dimethoxy-hexans wurden innerhalb von 90 Minuten unter $N_2$-Atmosphäre zu 70 ml einer unter Rückfluß siedenden 5%igen wäßrigen $H_2SO_4$ getropft, wonach das Reaktionsgemisch anschließend noch 3 Stunden unter Rückfluß zum Sieden erhitzt wurde.

Nach dem Abkühlen des Reaktionsansatzes wurde mit konz. Sodalösung neutralisiert, mit Essigsäureäthylester extrahiert, die organische Phase getrocknet und eingeengt. Es hinterblieben 21,3 g Rückstand. Bei der anschließenden Destillation wurden 10,3 g (IIIa) erhalten, das sich mit auf anderem Wege hergestelltem (IIIa) als identisch erwies. Schmelzpunkt: 68—70°C. Die Ausbeute an (IIIa) dem 2,5-Dimethyl-4-hydroxy-2,3-dihydro-furan-3-on betrug 45% d. Th., bezogen auf eingesetztes Roh-2,3; 4,5-Bis-epoxy-3,4-dimethoxyhexan.

b) Arbeitete man wie in Beispiel 9a) beschrieben, verwendete jedoch anstelle von 70 ml einer 5%igen wäßrigen Schwefelsäure die Lösung von 15 g Oxalsäuredihydrat in 400 ml Wasser, so erhielt man die Verbindung IIIa in etwa gleichen Ausbeuten.

c) Arbeitete man wie in Beispiel 9a) beschrieben, setzte jedoch anstelle von Roh-2,3; 4,5-Bis-epoxy-3,4-dimethoxy-hexan das gemäß Beispiel 5 erhaltene 2,3; 4,5-Bis-epoxy-3,4-diäthoxy-hexan ein, so erhielt man die Verbindung IIIa in etwa gleichen Ausbeuten wie in 9a).

Beispiel 10

15,5 g des gemäß Beispiel 8 erhaltenen Roh-2,3; 4,5-Bis-epoxy-3,4-dimethoxy-heptans wurden innerhalb von 2 Stunden unter $N_2$-Atmosphäre zu 100 ml einer unter Rückfluß siedenden 5%igen wäß-

rigen $H_2SO_4$ getropft, wonach das Reaktionsgemisch anschließend noch 3 Stunden unter Rückfluß zum Sieden erhitzt wurde. Bei der Aufarbeitung des Reaktionsgemisches analog Beispiel 9a) wurden 9,1 g Rohprodukt erhalten. Destillation lieferte 5,6 g (entsprechend 48% d. Th.) an dem Gemisch aus etwa 25% 2-Methyl-5-äthyl-4-hydroxy-2,3-dihydro-furan-3-on und etwa 75% 5-Methyl-2-äthyl-4-hydroxy-2,3-dihydro-furan-3-on von Siedepunkt Kp = 89 bis 91°C bei 0,1 mbar.

Beispiel 11

Zu 90 g einer 30%igen $NaOCH_3$-Lösung (0,5 Mol) wurde innerhalb von 2 Stunden bei Temperaturen von 0 bis 5°C die Lösung von 60 g (0,2 Mol) 2,5-Dibrom-octan-3,4-dion in 100 ml Methanol zugetropft. Anschließend wurde das Reaktionsgemisch noch 20 Stunden bei 0°C gerührt.

Bei einer Aufarbeitung analog Beispiel 1 erhielt man 2,3; 4,5-Bisepoxy-3,4-dimethoxy-octan in 70%iger Ausbeute. Der Siedepunkt beträgt bei 0,1 mbar 51 bis 52°C, der Brechungsindex $n_D^{25} = 1,4310$.

Beispiel 12

Arbeitete man wie im Beispiel 11 beschrieben, verwendete jedoch anstelle von 60 g 2,5-Dibrom-octan-3,4-dion 63 g (0,2 Mol) 2,5-Dibrom-nonan-3,4-dion, so erhielt man 2,3; 4,5-Bis-epoxy-3,4-dimethoxy-nonan in 71%iger Ausbeute. Kp bei 0,1 mbar = 61 bis 63°C; $n_D^{25} = 1,4334$.

Beispiel 13

Arbeitete man wie im Beispiel 11 beschrieben, verwendete jedoch anstelle von 60 g 2,5-Dibrom-octan-3,4-dion, 60 g 2,5-Dibrom-6-methyl-heptan-3,4-dion, so erhielte man das 2,3; 4,5-Bisepoxy-3,4-dimethoxy-6-methyl-heptan in 47%iger Ausbeute. Kp bei 0,01 mbar = 36°C, Brechungsindex $n_D^{25} = 1,4257$.

Beispiel 14

Arbeitete man wie in Beispiel 11 beschrieben, verwendete jedoch anstelle von 60 g 2,5-Dibrom-octan-3,4-dion 63 g 2,5-Dibrom-7-methyl-octan-3,4-dion, so erhielt man 2,3; 4,5-Bisepoxy-3,4-methoxy-7-methyl-octan in 58%iger Ausbeute. Kp bei 0,05 mbar = 50°C; $n_D^{25} = 1,4324$.

Beispiel 15

13 g (0,064 Mol) des gemäß Beispiel 11 erhaltenen 2,3; 4,5-Bisepoxy-3,4-dimethoxy-octans wurden entsprechend Beispiel 9a) mit 70 ml einer 5%igen wäßrigen $H_2SO_4$ umgesetzt. Man erhielt hierbei ein Gemisch (1:1) aus 2-Methyl-5-propyl und 2-Propyl-5-methyl-4-hydroxy-2,3-dihydro-furan-3-on in 48%iger Ausbeute. Kp des Gemisches bei 0,1 mbar = 85 bis 95°C.

Beispiel 16

15 g (0,07 Mol) des gemäß Beispiel 12 erhaltenen 2,3; 4,5-Bisepoxy-3,4-dimethoxy-nonans wurden entsprechend Beispiel 9a) mit 70 ml einer 5%igen wäßrigen $H_2SO_4$ umgesetzt. Man erhielt hierbei ein Gemisch (1:1) aus 2-Methyl-5-butyl- und 2-Butyl-5-methyl-4-hydroxy-2,3-dihydro-furan-3-on in 44%iger Ausbeute. Kp des Gemisches bei 0,1 mbar 101 bis 105°C.

Beispiel 17

9,5 g (0,047 Mol) des gemäß Beispiel 13 erhaltenen 2,3; 4,5-Bisepoxy-3,4-dimethoxy-6-methyl-heptans wurden entsprechend Beispiel 9a mit 70 ml einer 5%igen wäßrigen $H_2SO_4$ umgesetzt. Man erhielt hierbei das 2-Isopropyl-5-methyl-4-hydroxy-2,3-dihydro-furan-3-on in 35%iger Ausbeute. Kp bei 0,01 mbar = 120°C.

Beispiel 18

12 g (0,056 Mol) des gemäß Beispiel 14 erhaltenen 2,3; 4,5-Bisepoxy-3,4-methoxy-7-methyl-octans wurden entsprechend Beispiel 9a mit 70 ml einer 5%igen wäßrigen $H_2SO_4$ umgesetzt. Man erhielt hierbei ein Gemisch aus 2-Methyl-5-isobutyl- und 2-Isobutyl-5-methyl-4-hydroxy-2,3-dihydro-furan-3-on in 58%iger Ausbeute. Kp bei 0,1 mbar = 145 bis 150°C; $n_D^{20} = 1,4337$.

## Patentansprüche

1. Bis-epoxy-dialkoxy-alkane der allgemeinen Formel I

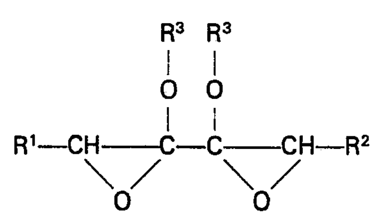

(I),

in der $R^1$ bis $R^3$ Alkyl mit 1 bis 4 C-Atomen bedeuten.

2. Verfahren zur Herstellung der neuen Bis-epoxy-dialkoxy-alkane der allgemeinen Formel I gemäß Anspruch 1, dadurch gekennzeichnet, daß man $\alpha,\alpha'$-Dihalogen-1,2-diketone der allgemeinen Formel II

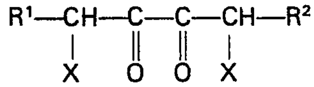

(II),

in der $R^1$ und $R^2$ die in Anspruch 1 angegebene Bedeutung haben und X für Cl oder Br steht bei Temperaturen von −20 bis +100°C, vorzugsweise 0 bis 25°C mit einem Alkalihydroxid oder einem Alkalialkoxid der Formel $R^3$—O—Me, in der Me für K, Na oder Li steht und einem Alkanol der Formel $R^3$—OH umsetzt.

3. Verwendung der neuen Bis-epoxy-dialkoxy-alkane der allgemeinen Formel I gemäß Anspruch 1 zur Herstellung von Aromastoffen der allgemeinen Formel III

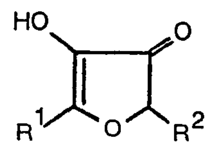

(III),

in der $R^1$ und $R^2$ die in Anspruch 1 angegebene Bedeutung haben, durch Erhitzen mit starken Mineralsäuren oder starken organischen Säuren auf Temperaturen von 60 bis 120°C.

4. Verfahren zur Herstellung von Aromastoffen der allgemeinen Formel III

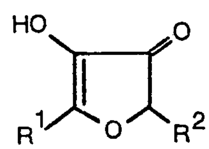

(III)

in der $R^1$ und $R^2$ Alkyl mit 1 bis 4 C-Atomen bedeuten, dadurch gekennzeichnet, daß man

A) $\alpha,\alpha'$-Dihalogen-1,2-diketone der allgemeinen Formel II

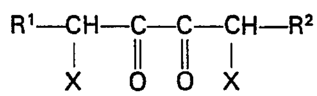

(II),

in der $R^1$ und $R^2$ die in Anspruch 1 angegebene Bedeutung haben und X für Cl oder Br steht bei Temperaturen von −20 bis +100°C mit einem Alkalihydroxid oder einem Alkalialkoxid der Formel $R^3$—O—Me, in der Me für K, Na oder Li steht und einem Alkanol der Formel $R^3$—OH umsetzt, und

B) die erhaltenen neuen Bis-epoxy-dialkoxy-alkane der allgemeinen Formel I

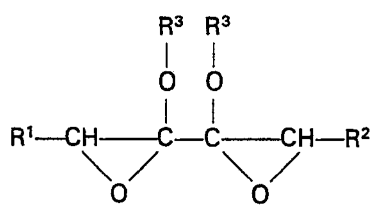

(I),

in der $R^1$ bis $R^3$ Alkyl mit 1 bis 4 C-Atomen bedeuten mit starken Mineralsäuren oder starken organischen Säuren auf Temperaturen von 60 bis 120°C erhitzt.

## Claims

1. A bis-epoxy-dialkoxy-alkane of the general formula I

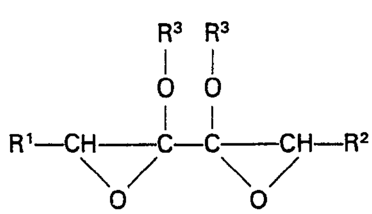

(I),

where $R^1$, $R^2$ and $R^3$ are alkyl of 1 to 4 carbon atoms.

2. A process for the preparation of a novel bis-epoxy-dialkoxy-alkane of the general formula I as claimed in claim 1, wherein an $\alpha,\alpha'$-dihalo-1,2-diketone of the general formula II

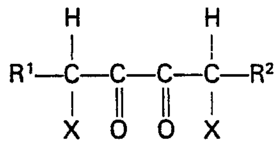

(II),

where $R^1$ and $R^2$ have the meanings given in claim 1 and X is Cl or Br, is reacted with an alkali metal hydroxide or an alkali metal alkoxide of the formula $R^3$—O—Me, where Me is K, Na or Li, and an alkanol of the formula $R^3$—OH, at from −20 to +100°C, preferably from 0 to 25°C.

3. Use of a novel bis-epoxy-dialkoxy-alkane of the general formula I as claimed in claim 1 for the preparation of an aroma material of the general formula III

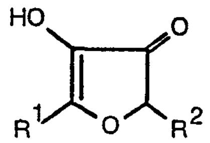

(III),

where $R^1$ and $R^2$ have the meanings given in claim 1, by heating with a strong mineral acid or a strong organic acid at from 60 to 120°C.

4. A process for the preparation of an aroma material of the general formula III

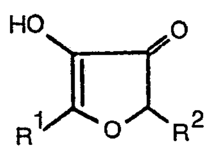

(III),

where $R^1$ and $R^2$ are alkyl of 1 to 4 carbon atoms, wherein

A) an $\alpha,\alpha'$-dihalo-1,2-diketone of the general formula II

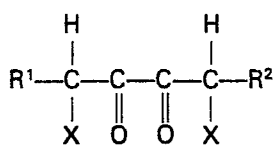

(II),

where $R^1$ and $R^2$ have the meanings given in claim 1 and X is Cl or Br, is reacted with an alkali metal hydroxide or an alkali metal alkoxide of the formula $R^3$—O—Me, where Me is K, Na or Li, and an alkanol of the formula $R^3$—OH, at from −20 to +100°C, and

B) the resulting novel bis-epoxy-dialkoxy-alkane of the general formula I

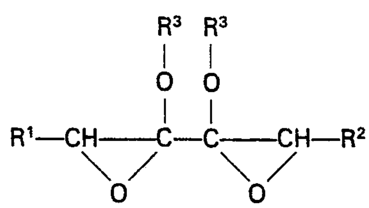

(I),

where $R^1$, $R^2$ and $R^3$ are alkyl of 1 to 4 carbon atoms, is heated with a strong mineral acid or a strong organic acid at from 60 to 120°C.

**Revendications**

1. Bis-époxy-dialcoxy-alcanes de formule générale I

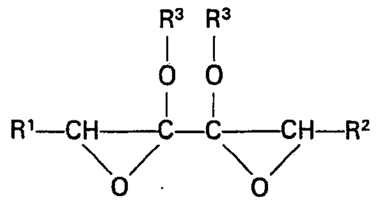

(I),

dans laquelle les symboles $R^1$ à $R^3$ représentent des groupes alkyle en C1—C4.

2. Procédé de préparation des nouveaux bis-époxy-dialcoxy-alcanes de formule générale I selon la revendication 1, caractérisé en ce que l'on fait réagir des alpha, alpha'-dihalogéno-1,2-dicétones de formule générale II

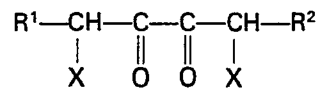

(II)

dans laquelle $R^1$ et $R^2$ ont les significations indiquées dans la revendication 1 et X représente Cl ou Br, à des températures allant de −20 à +100°C, de préférence de 0 à 25°C, avec un hydroxyde alcalin ou un alcoolate alcalin de formule $R^3$—O—Me dans laquelle Me représente K, Na ou Li, et un alcanol de formule $R^3$—OH.

3. Utilisation des nouveaux bis-époxy-dialcoxy-alcanes de formule générale I selon la revendication 1 dans la préparation de substances aromatiques de formule générale III

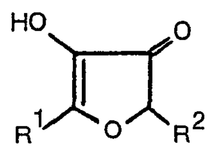

(III),

dans laquelle $R^1$ et $R^2$ ont les significations indiquées dans la revendication 1, par chauffage avec des acides minéraux forts ou des acides organiques forts à des températures de 60 à 120°C.

4. Procédé de préparation des substances aromatiques de formule générale III

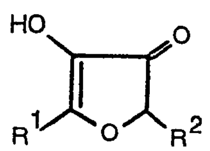

(III),

dans laquelle $R^1$ et $R^2$ représentent des groupes alkyle en C1—C4, caractérisé en ce que

A) on fait réagir des alpha-alpha'-dihalogéno-1,2-dicétone de formule générale II

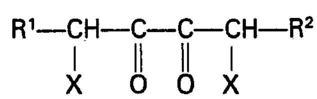

(II),

dans laquelle $R^1$ et $R^2$ ont les significations indiquées dans la revendication 1, et X représente Cl ou Br, à des températures allant de −20 à +100°C, avec un hydroxyde alcalin ou un alcoolate alcalin de formule $R^3$—O—Me dans laquelle Me représente K, Na ou Li, et un alcool de formule $R^3$—OH, et

B) on chauffe les nouveaux bis-époxy-dialcoxy-alcanes obtenus de formule générale I

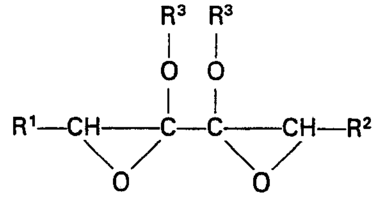

(I),

dans laquelle les symboles $R^1$ à $R^3$ représentent des groupes alkyle en C1—C4, avec des acides minéraux forts ou des acides organiques forts à des températures de 60 à 120°C.